(19) Europäisches Patentamt
European Patent Office
Office européen des brevets

(11) Publication number: 0 073 146
B1

(12) **EUROPEAN PATENT SPECIFICATION**

(45) Date of publication of patent specification: **11.06.86**

(51) Int. Cl.$^4$: **G 03 C 7/34** // C07C127/19

(21) Application number: **82304386.4**

(22) Date of filing: **19.08.82**

# (54) Method and material for the formation of silver halide color photographic image.

(30) Priority: **20.08.81 JP 131312/81**

(43) Date of publication of application:
**02.03.83 Bulletin 83/09**

(45) Publication of the grant of the patent:
**11.06.86 Bulletin 86/24**

(84) Designated Contracting States:
**DE FR GB**

(56) References cited:
**EP-A-0 028 099**
**EP-A-0 067 689**
**FR-A-2 125 969**
**US-A-3 446 622**
**US-A-3 880 661**

**The file contains technical information submitted after the application was filed and not included in this specification**

(73) Proprietor: **KONISHIROKU PHOTO INDUSTRY CO. LTD.**
**No. 26-2, Nishishinjuku 1-chome Shinjuku-ku**
**Tokyo 160 (JP)**

(72) Inventor: **Kato, Katsunori**
**2-12-21 Nakanosanoh-machi**
**Hachioji-shi Toyko (JP)**
Inventor: **Sato, Ryosuke**
**5995 Hino**
**Hino-shi Tokyo (JP)**

(74) Representative: **Ellis-Jones, Patrick George Armine et al**
**J.A. KEMP & CO. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 073 146 B1

**Description**

The present invention relates to a silver halide color photographic light-sensitive material containing a novel cyan dye image forming coupler. A color photographic image is normally formed by the oxidation coupling reactions effected inside a silver halide emulsion between the oxidization product of a color developing agent produced during the reduction of the exposed silver halide particles by an aromatic primary amine color developing agent and couplers, to form yellow, magenta and cyan dyes.

Typical couplers for use in the formation of cyan dyes are phenols and naphthols. These fundamental requirements which have conventionally been considered to be essential for photographic characteristics, particularly of phenol couplers are that the dye formed from the coupler must have satisfactory spectral absorption characteristics; i.e. have a sharp-cut filtering effect with no absorption in the green region of the spectrum; the dye formed must have sufficient durability against light, heat, moisture, and the like; the coupler should possess excellent color developability; i.e. have a sufficiently color-developable sensitivity and color developed density; and no loss of dye formation is to occur even in continuous processing during which the bleaching bath or bleach-fixing bath composed principally of the ferric salt of EDTA becomes exhausted.

From the anti-pollution point of view, benzyl alcohol frequently added to the color developing bath is desirably removed from the bath, but the addition of the alcohol to the color developing bath is essential for sufficient color developability; this has been a serious problem. The deterioration in color developability if the use of benzyl alcohol is eliminated is particularly noticeable in the case of cyan couplers. For this reason, the development of a phenol cyan coupler which gives a benzyl alcohol-free, high color developability is an urgent need.

Many attempts have hitherto been made to satisfy the above-described requirements, but so far no cyan couplers have been found that can satisfy all the characteristics required as mentioned above.

For example, 6-[α-(2,4-di-t-amyl phenoxy) butaneamide]-2,4-di-chloro-3-methyl phenol described in U.S. Patent No. 2,801,171 possesses excellent light resistance, but is disadvantageous because of its unsatisfactory heat resistance, and, in addition, because of the loss of the resulting dye in an exhausted bleach-fixing solution. Further, its color developability depends largely upon benzyl alcohol, so that it is difficult to eliminate benzyl alcohol from the color developing solution. 2-Heptafluorobutaneamide-5-[α-(2,4-di-t-amylphenoxy) hexaneamide]phenol described in U.S. Patent No. 2,895,826 is excellent in that there is little loss of dye in an exhausted bleach-fixing bath and also it has good heat resistance, but is disadvantageous from the viewpoint of color developability and light resistance. The coupler described in Japanese Patent Publication Open to Public Inspection (hereinafter referred to as Japanese Patent O.P.I. Publication) No. 109630/1978 also poses a problem in relation to light resistance as well as regards the removal of benzyl alcohol. Further, those phenol cyan couplers described in U.S. Patent No. 3,839,044, Japanese Patent O.P.I. Publication No. 37425/1972, Japanese Patent Examined Publication No. 36894/1973, Japanese Patent O.P.I. Publication Nos. 10135/1975, 117422/1975, 108841/1975, 120334/1975, and the like are unsatisfactory in relation to the removal of benzyl alcohol. Those phenol couplers having a ureido group in the two position thereof are described in British Patent No. 1,011,940 and U.S. Patent Nos. 3,446,622, 3,996,253, 3,758,308 and 3,880,661, but cyan dyes formed from these couplers have broad spectral absorptions whose absorption maxima are located in relatively shorter wavelengths in the red region of the spectrum, so that they are not acceptable for color reproduction. The phenol coupler having a ureide group in the two position thereof described in Japanese Patent O.P.I. Publication No. 65134/1981 is one which is relatively good as regards its absorption in the green region in the spectrum, but is still not satisfactory in respect of other characteristics. European Specification 0028099 discloses 2-phenylureido-5-acylamino phenol cyan couplers in which the phenyl group is substituted by cyano while our earlier filed European Specification 0073146 discloses such couplers in which the phenyl group is substituted by a variety of substituents but not those used in the present invention.

As a result of prolonged studies we have found, according to the present invention, that a coupler having the formula given below can satisfy all the requirements essential for phenol cyan couplers.

The cyano couplers of the present invention have the formula:

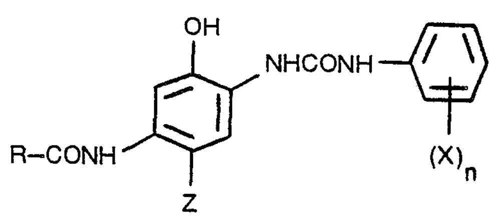

(I)

wherein R is a substituted or unsubstituted alkyl group, generally having from 1 to 20 carbon atoms (preferably a phenoxyalkyl), a subsdtituted or unsubstituted aryl group (preferably a phenyl group) or a heterocyclic group (e.g. a nitrogen-containing heterocyclic group such as piperidine or pyrrolidine); X is a substituted or unsubstituted carbonamido, for example a substituted or unsubstituted alkyl carbonamide group, generally having from 1 to 20 carbon atoms, or benzoylamide group, sulfonamido, for example a

substituted or unsubstituted alkyl sulfonamide group (which can be cyclic), generally having from 1 to 20 carbon atoms, or a benzene sulfonamide group, or succinimido group; Z represents hydrogen or an atom or group capable of being eliminated during the coupling reaction of the coupler with the oxidization product of a color developing agent, e.g a halogen atom (such as chlorine, bromine or fluorine), an aryloxy, carbamoyloxy, carbamoylmethoxy, acyloxy, sulfonamido or siccinic acid imido group in which an oxygen or nitrogen atom is directly attached to the coupling position thereof, further examples of Z being described in U.S. Patent No. 3,471,563, Japanese Patent O.P.I. Publication No. 37425/1972, Japanese Patent Examined Publication No. 36894/1973, Japanese Patent O.P.I. Publication Nos. 10135/1975, 117422/1975, 130441/1975, 108841/1976, 120334/1975, 18315/1977, 52423/1978 and 105226/1978; and n is an integer from 1 to 3, such that when n is 2 or 3, each X can be the same or different.

Examples of cyan couplers of the present invention are given below:

(3)

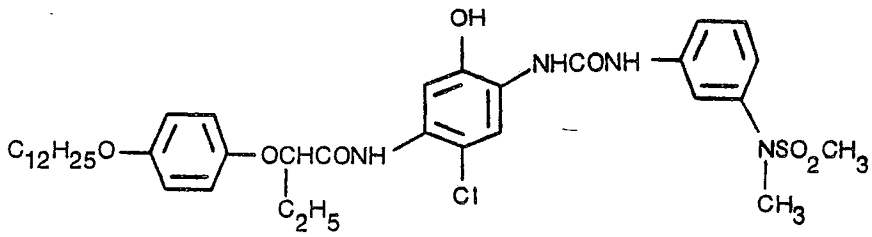

(7)

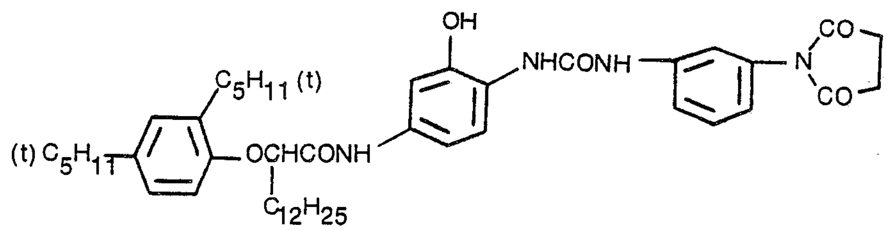

(16)

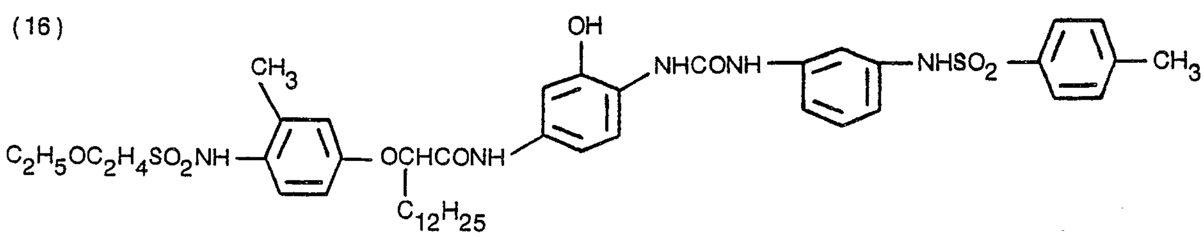

(17)

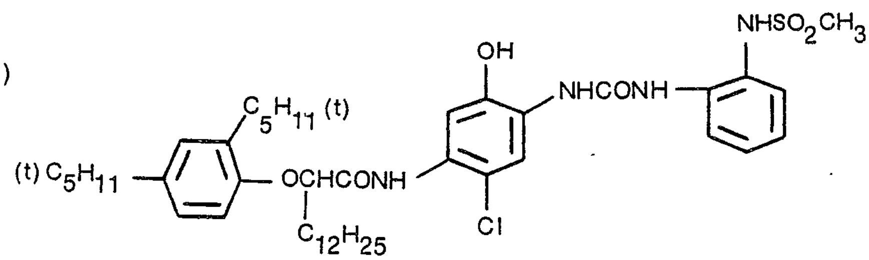

(20)

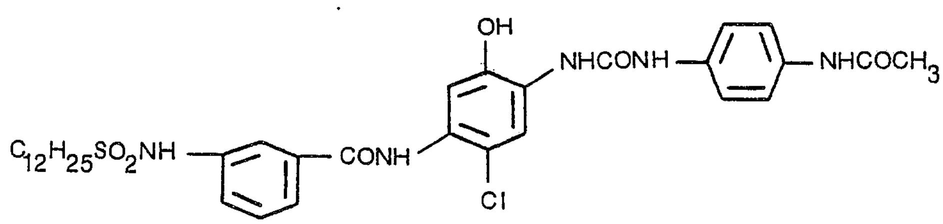

The following are typical ways of synthesizing the couplers of the present invention:

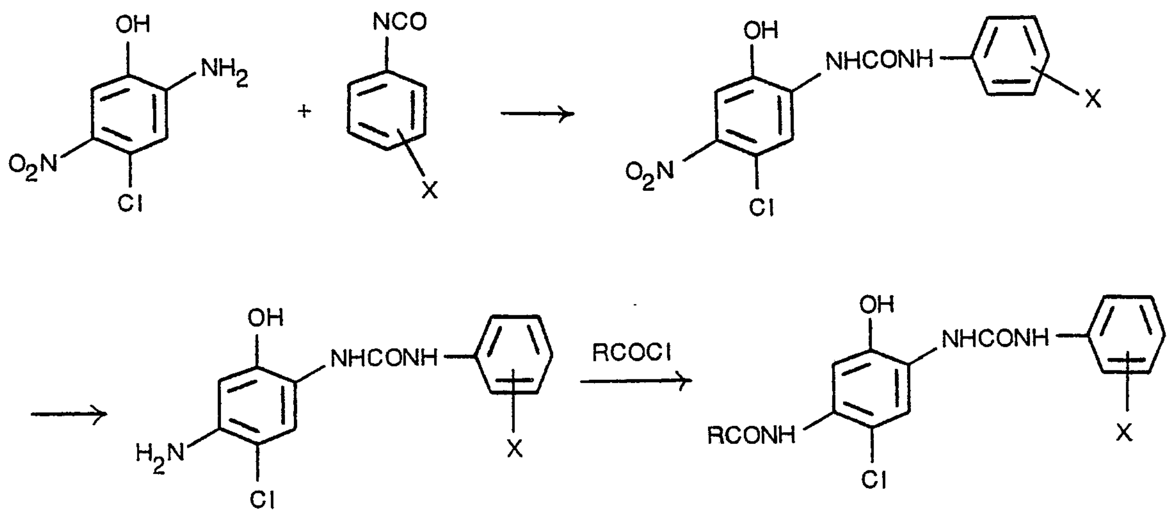


The couplers can be obtained in a similar manner to the following illustrative examples.

Synthesis of 4-chloro-5-[3-{3(hexadecyl-sulfoneamide)phenoxy}butaneamide]-2-(2,4-dimethoxyphenyl ureido)phenol.

18.9 g of 2-amino-4-chloro-5-nitrophenol were dispersed into 200 ml of toluene, and to the mixture were added with stirring at room temperature 100 ml of a toluene solution containing 18.0 g of 2,4-dimethoxyphenylisocyanate. The resulting reaction mixture was boiled with refluxing for 1 hour. After cooling to room temperature, the deposited crystals were collected by filtration and washed with methanol, whereby 31 g of yellow crystals were obtained.

18 g of 4-chloro-2-(2,4-dimethoxy-phenyl) ureide)-5-nitrophenol were added to 600 ml of alcohol, and the solution was subjected to hydrogenation by the addition of a palladium-carbon catalyst under normal pressure. After the consumption of the theoretical amount of hydrogen, the mixture was heated and the palladium-carbon catalyst was filtered off while hot. The filtrate was concentrated under reduced pressure, and to the residue was added n-hexane to deposit crystals which were then collected by filtration and then washed, thereby obtaining 14 g of a white solid.

3.4 g of 5-amino-4-chloro-2-(2,4-dimethoxyphenyl ureide) phenol were added to 100 ml of an acetonitrile solution containing 0.9 ml of pyridine, and to the solution, with stirring at room temperature were added dropwise 50 ml of an acetonitrile solution containing 5.5 g of 3-(m-hexadecyl sulfonamide phenoxy)butyloyl chloride. After completion of the addition, the reaction of the mixture took place for one hour, and then the mixture was poured into water. The resulting oily product was extracted using ethyl acetate. The thus produced oily substance was refined with silica gel by column chromatography, and finally solidified using n-hexane, thus obtaining 3.7 g of a white solid. The structure of the product was ascertained through the procedures of nuclear magnetic resonance and mass spectrometry.

Synthesis of 4-chloro-5-(m-hexadecyloxycarbonyl benzoylamide)-2-(2,4-di-methoxyphenyl ureide)phenol.

3.4 g of 5-amino-4-chloro-2-(2,4-dimethoxyphenyl ureide) phenol were added to a mixed solution of 100 ml of acetonitrile and 0.9 ml of pyridine, and to the solution, with stirring at room temperature, were little by little added 4.5 g of m-hexadecyl-oxycarbonyl-benzoyl chloride. After completion of the addition, the reaction took place for one hour, and then the reaction product was poured into water. The resulting oily product was extracted with ethyl acetate, was subjected to silica gel-column chromatography, and was then solidified by use of n-hexane, whereby 2.7 g of a white solid were obtained. The structure of the thus obtained product was ascertained through the procedures of nuclear magnetic resonance and mass spectrometry.

To the cyan dye forming coupler of the present invention, ordinary methods and techniques for ordinary cyan dye forming couplers may be applied. Typically, the coupler is incorporated into a silver halide emulsion, and this emulsion is coated on a base to form a photograhic element.

The photographic element may be either a monochromatic or a multicolor element. In the case of a multicolor element, the cyan dye forming coupler is normally incorporated into a red-sensitive emulsion. However, an unsensitized emulsion or each component unit provided with a dye image forming component having its sensitivity in the relevant one of the three primary color regions of the spectrum may be composed of either a single emulsion layer or multi-coated emulsion layers. The layers of the element, including each image forming component unit nay be arranged in various orders as is well-known to those skilled in the art. A typical multicolor photographic element comprises a base provided thereon with a cyan dye image forming component unit composed of at least one red-sensitive silver halide emulsion layer

containing at least one cyan dye forming coupler (at least one of the cyan dye forming couplers being a coupler of the present invention), a magenta dye image forming component unit composed of at least one green-sensitive silver halide emulsion layer containing at least one magenta dye forming coupler, and a yellow dye image forming component unit composed of at least one blue-sensitive silver halide emulsion layer containing at least one yellow dye forming coupler. The element may be provided with additional layers such as filter layers, interlayers, a protective layer and a subbing layer.

The incorporation of the coupler of the present invention into an emulsion layer may be performed in a conventionally known manner. For example, a single coupler or couplers in combination in the present invention may be dissolved in a high boiling organic solvent whose boiling point is not less than 175°C such as tricresyl phosphate or dibutyl phthalate or a low boiling solvent such as butyl acetate or butyl propionate or, if necessary, a mixture of such solvents, and the solution obtained is subsequently mixed with an aqueous gelatin solution containing a surfactant. The resulting mixture is emulsified by means of a highspeed rotary mixer or a colloid mill, and then a silver halide is added to obtain a silver halide emulsion intended for use in the present invention. The amount of the coupler of the present invention in the silver halide emulsion of the present invention is normally from 0.07 to 0.7 mol, and preferably from 0.1 to 0.4 mol, per mol of the silver halide.

Silver halides which can be used in the silver halide emulsion of the present invention are those used in ordinary silver halide emulsions and silver bromide, silver chloride, silver iodobromide, silver chlorobromide and silver chloroiodobromide.

Silver halide emulsions that may constitute the silver halide emulsion of the present invention may be prepared by various methods such as, in addition to ordinary methods, the method for the preparation of the so-called conversion type emulsion, as described in Japanese Patent Examined Publication No. 7772/1971, where an emulsion of silver halide particles at least a part of which has a larger solubility than that of silver bromide is formed, and at least a part of the particles is then converted into silver bromide or silver iodobromide; the method for the preparation of Lippman type emulsion comprising a fine-grained silver halide having a mean particle diameter of not more than 0.1 μ; or other equivalent methods.

The silver halide emulsion of the present invention may be chemically sensitized by using, singly or in combination, a sulfur sensitizer such as aryl-thio-carbamide, thiourea or cystine; an active or inert selenium sensitizer; a reduction sensitizer such as a stannous salt or a polyamine, a noble metal sensitizer such as a gold sensitizer examples of which include potassium aurothiocyanate, potassium chloroaurate and 2-aurosulfobenzothiazol-methyl chloride; or a water-soluble salt sensitizer such as a ruthenium, rhodium or iridium salt, examples of which include ammonium chloropalladate, potassium chloroplatinate and sodium chloropalladite.

The silver halide emulsion of the present invention may contain various known photographic additives such as those described in Research Disclosure Item No. 17643 (December 1978).

In order to render the silver halide of the present invention sensitive to the necessary wavelength region to which any red-sensitive emulsion is to be sensitive, the silver halide can be spectrally sensitized by an appropriately selected sensitizer. For the spectral sensitization, there may be used, singly or in combination, various spectral sensitizers, typical examples of which include cyanine dyes, merocyanine dyes and combined cyanine dyes, as described in, e.g. U.S. Patent Nos. 2,269,234, 2,270,378, 2,442,710, 2,454,620 and 2,776,280.

A color developing bath intended for use in the present invention is one that contains as the principal component thereof an aromatic primary amine color developing agent, examples of which include p-phenylenediamines such as diethyl-p-phenylenediamine hydrochloride, monomethyl-p-phenylenediamine hydrochloride, dimethyl-p-phenylenediamine hydrochloride, 2-amino-5-diethylaminotoluene hydrochloride, 2-amino-5-(N-ethyl-N-dodecylamino)-toluene, 2-amino-5-(N-ethyl-N-β-methanesulfoneamide ethyl)aminotoluene sulfate, 4-(N-ethyl-N-β-methanesulfoneamide ethylamino)aniline, 4-(N-ethyl-N-β-hydroxyethylamino) aniline and 2-amino-5-(N-ethyl-N-β-methoxyethyl)aminotoluene.

Development is followed by the usual process such as bleaching, fixing or bleach-fixing for the removal of the silver and silver halide, washing and then drying.

The present invention is further illustrated in the following Examples.

Example 1

0.03 mole of each of one of the couplers of the present invention as shown in Table 1 and one of couplers (A), (B) and (C) given below was added to a liquid prepared by mixing one part of dibutyl phthalate in the same amount by weight as that of the coupler mixture with three parts of ethyl acetate, and completely dissolved therein by stirring with heating to 60°C. The resulting solution was added to an aqueous gelatin solution containing Alkanol B (alkyl-naphthalene sulfonate, Registered Trade Mark of DuPont) and emulsified by means of a colloid mill to thereby prepare coupler-dispersed liquids. Each of the resulting coupler-dispersed liquids was added to a silver chlorobromide emulsion containing 0.1 mole of silver, and each of the thus produced emulsions was coated on a polyethylene-laminated paper and then dried, whereby six different stable layer silver halide color photoagraphic light-sensitive materials were obtained (Sample Nos. (1) to (6)).

Control Coupler (A)

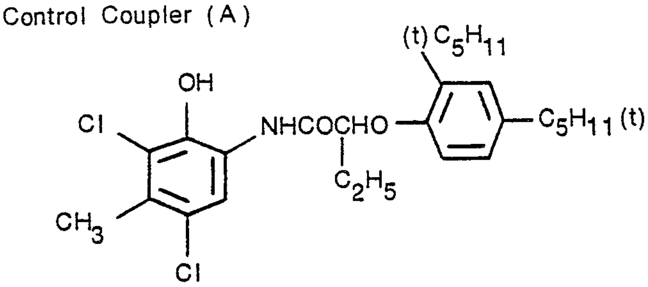

Control Coupler (B)

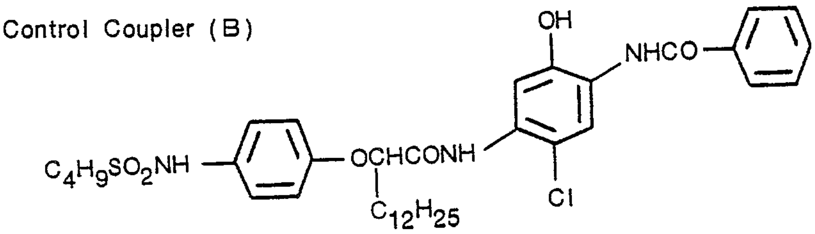

Control Coupler (C)

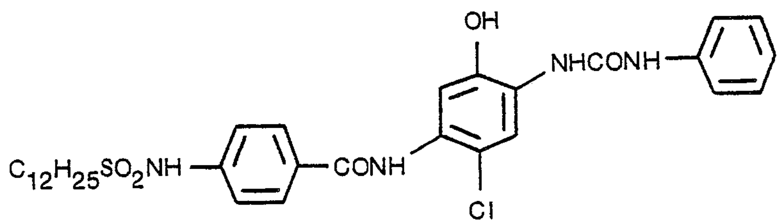

These samples were exposed through an optical wedge to light using the normal sensitometric procedure, and then processed using the following steps, the color developing being carried out in one of two different composition baths: one with added benzyl alcohol (color developing bath composition 1) and the other without benzyl alcohol (color developing bath composition 2).

Processing steps:

| Processing steps (at 30°C) | Processing period |
|---|---|
| Color developing | 3 min. 30 sec. |
| Bleach-fixing | 1 min. 30 sec. |
| Washing | 2 min. |

The respective bath compositions are as follows:

Color developing bath composition 1:

| | | |
|---|---|---|
| 4-amino-3-methyl-N-ethyl-N-(β-methane-sulfoneamide ethyl)-aniline sulfate | 5.0 | g |
| Benzyl alcohol | 15.0 | ml. |
| Sodium hexametaphosphate | 2.5 | g |
| Anhydrous sodium sulfite | 1.85 | g |
| Sodium bromide | 1.4 | g |
| Potassium bromide | 0.5 | g |
| Borax | 39.1 | g |

Water to make 1 liter. Add sodium hydroxide to adjust the pH to 10.30.

Color developing bath composition 2:

| | |
|---|---|
| 4-amino-3-methyl-N-ethyl-N-(β-methane-sulfoneamide ethyl)-aniline sulfate | 5.0 g |
| Sodium hexametaphosphate | 2.5 g |
| Anhydrous sodium sulfite | 1.85 g |
| Sodium bromide | 1.4 g |
| Potassium bromide | 0.5 g |
| Borax | 39.1 g |
| Water to make 1 liter. Add sodium hydroxide to adjust the pH to 10.30. | |

Bleach-fixing bath composition:

| | |
|---|---|
| Iron-ammonium ethylenediamine tetraacetate | 50 g |
| Ammonium sulfite (40% aqueous solution) | 50 ml |
| Ammonium thiosulfate (70% aqueous solution) | 140 ml |
| Aqueous ammonia (28% solution) | 20 ml |
| Ethylenediamine tetraacetate | 4 g |
| Water to make 1 liter | |

The respective samples thus processed were measured for their photographic characteristics. The results are as shown in Table 1. The relative photographic speeds shown in Table 1 are those relative to the maximum speed taken as 100 for the sample containing Control coupler (A) processed in the color developing bath composition 1.

TABLE 1

| Sample No. | Coupler used | Color developing bath composition 1 | | Color developing bath composition 2 | |
|---|---|---|---|---|---|
| | | Relative speed | Maximum density | Relative speed | Maximum density |
| 3 | Exemplified compound — 20 | 100 | 2.20 | 85 | 1.90 |
| 4 | Control coupler — A | 100 | 2.20 | 50 | 1.40 |
| 5 | Control coupler — B | 95 | 1.95 | 61 | 1.50 |
| 6 | Control coupler — C | 90 | 1.80 | 55 | 1.51 |

As is apparent from Table 1, the sample obtained using the coupler of the present invention, regardless of whether benzyl alcohol is used or not, is found to possess excellent speed and maximum density.

As a result of measuring the spectra of the color developed, the dye formed from the coupler of the present invention was found to possess excellent color purity having absorption wavelength maximum in the relatively longer wavelength portion and little absorption in the shorter wavelength portion in the red region of the spectrum.

Example 2

Samples obtained in the same manner as in Example 1 were tested for their light resistance, heat resistance and moisture resistance. The results of these tests are as shown in Table 2.

TABLE 2

| Sample No. | Coupler used | Color developing bath composition 1 | | | Color developing bath composition 2 | | |
|---|---|---|---|---|---|---|---|
| | | Light resistance | Heat resistance | Moisture resistance | Light resistance | Heat resistance | Moisture resistance |
| 9 | Exemplified compound — 20 | 85 | 100 | 93 | 85 | 97 | 93 |
| 10 | Control coupler — A | 90 | 45 | 60 | 85 | 47 | 63 |
| 11 | Control coupler — B | 60 | 90 | 92 | 60 | 95 | 90 |
| 12 | Control coupler — C | 55 | 90 | 92 | 55 | 95 | 90 |

The values given in the light resistance columns of the table are the relative values of the residual densith obtained after exposing the respective resulting images to the light of a xenon fadometer for a duration of 300 hours to the corresponding sensitivity values regarded as 100 of the same samples prior to exposure to the light. The values in the moisture resistance columns of the table are the relative residual density values after aging the samples at 60°C/70% RH for a duration of three weeks to the corresponding density values regarded as 100 of the samples before the aging. The heat resistance values are the residual densities of the samples after being subjected to aging at 77°C over a period of three weeks relative to the corresponding values regarded as 100 of the samples before the aging (the initial density being taken as 1.0).

As is apparent from Table 2, Control Coupler (A) shows excellent resistance to light, but has poor heat resistance, while Control Couplers (B) and (C) possess excellent heat resistance; but have a poor light resistance.

On the other hand, Exemplified Coupler (20) is found to have excellent characteristics in every aspect.

## Example 3

Each of Samples (3) to (6) obtained in Example 1, after being exposed through an optical wedge to light, was processed in the bath of color developing bath composition 1 in Example 1, and then a part of each of the developed samples was processed in the foregoing bleach-fixing bath while the other part was processed in an exhausted bath-simulated bleach-fixing solution of the following composition to thereby examine possible discolorations of the formed cyan dyes due to the exhaustion of a bleach-fixing bath.

Bleach-fixing bath composition:

| | |
|---|---|
| Iron-ammonium ethylenediamine tetraacetate | 50g |
| Ammonium sulfite (40%) aqueous solution) | 50 ml |
| Ammonium thiosulfate (70% aqueous solution) | 140 ml |
| Aqueous ammonia (28% solution) | 20 ml |
| Ethylenediamine tetraacetate | 4 g |
| Sodium hydrosulfite | 5 g |
| Water to make 1 liter | |

Each of the thus processed samples was measured for the maximum reflection density of the formed cyan dye thereof. The results are as shown in Table 4.

The residual rate of the dye in the maximum density area was determined by the following formula:

$$\text{Residual rate of dye} = \frac{\text{Exhausted bleach-fixing bath processing}}{\text{Fresh bleach-fixing bath processing}} \times 100$$

TABLE 4

| Coupler used | Fresh BF* processing | Exhausted BF* processing | Residual rate of dye |
|---|---|---|---|
| Exemplified compound — 20 | 2.20 | 2.10 | 95 |
| Control coupler — A | 2.20 | 1.43 | 65 |
| Control coupler — B | 1.95 | 1.93 | 99 |
| Control coupler — C | 1.80 | 1.72 | 96 |

Note: * "BF" stands for Bleach-Fixing bath.

From Table 4 is can be seen that the sample containing the coupler of the present invention shows little discoloration of the formed cyan dye in the exhausted bleach-fixing bath as compared to those containing the control couplers.

**Claims**

1. A method for the formation of a silver halide color photographic dye image characterised by forming the dye image in the presence of a phenol cyan coupler which has the formula:

Formula (I)

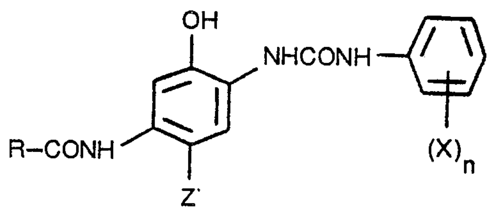

(I)

wherein R is an optionally substituted alkyl, aryl or heterocyclic group; X is an unsubstituted or substituted carbonamido, succinimido or sulfonamido group; Z is a hydrogen atom or an atom or group capable of being eliminated during coupling with the oxidization product of a color developing agent; and n is an integer from 1 to 3, each said X being the same or different when n is 2 or 3.

2. A method according to claim 1, wherein Z is a halogen atom, an aryloxy group, a carbamoyloxy group, a carbamoylmethoxy group, an acyloxy group, a sulfonamido group or a succinic acid imido group, in which an oxygen or nitrogen atom is directly attached to the coupling position of the coupler.

3. A method according to claim 1 or 2 wherein X is a carbonamido or sulfonamido group.

4. A method according to claim 3 wherein the carbonamido group is an alkyl carbonamido group or a benzoylamido group.

5. A method according to claim 3 wherein the sulfonamido group is an alkylsulfonamido group or a benzenesulfonamido group.

6. A light-sensitive silver halide photographic material comprising a phenol cyan coupler as defined in any one of claims 1 to 5.

7. A phenol cyan coupler which has the formula (I) as defined in claim 1.

8. A phenol cyan coupler according to claim 7 which is as defined in any one of claims 2 to 5.

**Patentansprüche**

1. Verfahren zur Bildung eines photographischen Silberhalogenid-Farbphotos, dadurch gekennzeichnet, daß das Farbbild in Gegenwart eines Phenolcyankupplers der Formel I

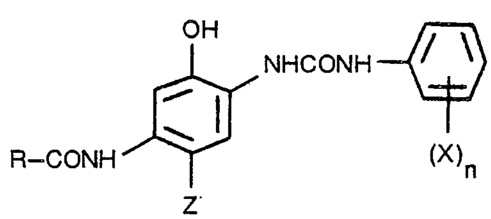

(I)

worin R ein gegebenenfalls substituierter Alkyl-, Aryl- oder heterocyclischer Rest ist, X ein unsubstituierter oder substituierter Carbonamido-, Succinimido- oder Sufonamidorest ist, Z ein Wasserstoffatom oder ein Atom oder ein Rest ist, der (das) während der Kupplung mit dem Oxidationsprodukt eines Farbentwicklungsmittels abgespalten werden kann, und n eine ganze Zahl von 1 bis 3 bedeutet, wobei jedes X gleich oder unterschiedlich ist, wenn n 2 oder 3 ist, gebildet wird.

2. Verfahren nach Anspruch 1, worin Z ein Halogenatom, ein Aryloxyrest, ein Carbamoyloxyrest, ein Carbamoylmethoxyrest, ein Acyloxyrest, ein Sulfonamidorest oder ein Succinsäureimidorest ist, worin ein Sauerstoff- oder Stickstoffatom direkt an die Kupplungsstelle des Kupplers gebunden ist.

3. Verfahren nach Anspruch 1 oder 2, worin X ein Carbonamido- oder ein Sulfonamidorest ist.

4. Verfahren nach Anspruch 3, worin der Carbonamidorest ein Alkylcarbonamidorest oder ein Benzoylamidorest ist.

5. Verfahren nach Anspruch 3, worin der Sulfonamidorest ein Alkylsulfonamidorest oder ein Benzolsulfonamidorest ist.

6. Lichtempfindliches photographisches Silberhalogenidmaterial, welches einen Phenolcyankuppler gemäß der Definition in einem der Ansprüche 1 bis 5 enthält.

7. Phenolcyankuppler gemäß Formel (I) nach der Definition in Anspruch 1.

8. Phenolcyankuppler nach Anspruch 7 gemäß der Definition in einem der Ansprüche 2 bis 5.

**Revendications**

1. Procédé de formation d'une image photographique en couleurs d'halogénure d'argent, caractérisé en ce qu'on forme l'image de colorant en présence d'un agent de copulation cyan phénolique qui répond à la formule:

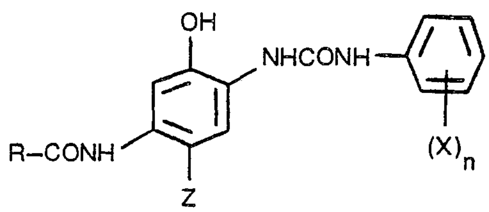

(I)

dans laquelle R est un groupe alkyle, aryle ou hétérocyclique éventuellement substitué; X est un groupe carbamido, succinimido ou sulfamido non substitué ou substitué; Z est un atome d'hydrogène ou un atome ou groupe susceptible d'être éliminé au cours de la copulation avec le produit d'oxydation d'un agent de développement en couleur; et n est un entier de 1 à 3, chacun de ces X étant identique ou différent lorsque n est 2 ou 3.

2. Procédé selon la revendication 1, dans lequel Z est un atome d'halogène, un groupe aryloxy, un groupe carbamoyloxy, un groupe carbamoylméthoxy, ou un groupe acyloxy, un groupe sulfamido ou un groupe succinimido, dans lequel un atome d'hydrogène ou d'azote est directement fixé à la position de copulation du copulant.

3. Procédé selon les revendications 1 ou 2, dans lequel X est un groupe carbamido ou sulfamido.

4. Procédé selon la revendication 3, dans lequel le groupe carbamido est un groupe alkylcarbamido ou un groupe benzoylamido.

5. Procédé selon la revendication 3, dans lequel le groupe sulfamido est un groupe alkylsulfamido ou un groupe benzènesulfamido.

6. Matière photographique dlhalogénure d'argent photo-sensible, comprenant un agent de copulation cyan phénolique tel que défini dans l'une quelconque des revendications 1 à 5.

7. Agent de copulation cyan phénolique qui répond à la formule I tel que défini dans la revendication 1.

8. Agent de copulation cyan phénolique selon la revendication 7, qui est tel que défini dans l'une quelconque des revendications 2 à 5.